# EUROPÄISCHE PATENTSCHRIFT

(11) **EP 4 085 957 B1**
(45) Veröffentlichungstag und Bekanntmachung des Hinweises auf die Patenterteilung: **18.06.2025**
(21) Anmeldenummer: 22020260.0
(22) Anmeldetag: 20.03.2020
(51) Int. Cl.: A61M 16/06

(54) **KOPFBÄNDERUNG FÜR EIN PATIENTENINTERFACE**
HEAD HARNESS FOR A PATIENT INTERFACE
HARNAIS DE TÊTE POUR UNE INTERFACE PATIENT

(30) Priorität: 27.03.2019 DE 102019107902
(43) Veröffentlichungstag der Anmeldung: 09.11.2022
(62) Teilanmeldung aus: 20000123.8
(73) Patentinhaber: Löwenstein Medical Technology S.A., 2557 Luxembourg (LU)
(72) Erfinder: Eifler, Martin, 25348 Glückstadt (DE)
(74) Vertreter: Löwenstein Medical IP

(56) Entgegenhaltungen:
- WO-A1-2010/066004
- WO-A1-2014/175753
- WO-A1-2016/075658
- WO-A1-2021/137765
- CH-B1- 700 500
- US-A1- 2010 000 544
- US-A1- 2015 283 348
- US-A1- 2016 030 696
- US-A1- 2016 074 614
- US-A1- 2016 143 766

## Beschreibung

Die vorliegende Erfindung betrifft eine Kopfbänderung für ein Patienteninterface wobei die Kopfbänderung aus zumindest einem Stoffstrang ausgebildet ist, wobei der Stoffstrang zumindest bereichsweise einen Verstärkungsbereich aufweist.

Aus dem Dokument US 2017 189 636 A1 ist eine Kopfbedeckung für eine Atemmaske mit einer Riemenanordnung mit vorderen Riemenabschnitten, die über den Wangen eines Patienten angeordnet werden können, bekannt, wobei deren vordere Enden zur Befestigung an einem Luftweg-Schnittstellenabschnitt ausgebildet sind. Die vorderen Riemenabschnitte weisen dünne Versteifungsvorrichtungen auf, die eingerichtet sind, die vorderen Riemenabschnitte zu versteifen, zu stabilisieren, zu positionieren und/oder zu krümmen. Die Versteifungsvorrichtungen helfen, die Kopfbedeckung in einer offenen Form zu halten, wenn sie nicht verwendet wird, um einem Patienten das Anlegen der Maske zu erleichtern. Die Versteifungsvorrichtungen sind von außen auf den vorderen Riemenabschnitten angeordnet.

US 2015/0283348 A1 beschreibt eine elastische Kopfbedeckung für ein Patienteninterface mit einem Riemenelement umfassend ein Basiselement mit einem Verstärkungsabschnitt an dessen Oberseite sowie vier verstärkten Armabschnitten aus einem unelastischen Material, wobei das Patienteninterface eine nasale oder orale Maske umfassen kann. Die Kopfbedeckung kann auch ohne Stirnstütze in Position gehalten werden.

Weitere Beispiele für Kopfbänderungen werden in US 2010/0000544 A1 und WO 2010/066004 A1 beschrieben.

Es ist Aufgabe der vorliegenden Erfindung, eine Bänderung bereitzustellen, die zumindest eine bereichsweise Stabilisierung der Kopfbänderung ermöglicht und gleichzeitig einen hohen Tragekomfort für einen Patienten bietet.

Diese Aufgabe wird durch eine Kopfbänderung gemäß dem Anspruch 1 gelöst.

Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Die vorliegende Erfindung betrifft eine Kopfbänderung für ein Patienteninterface wobei die Kopfbänderung aus zumindest einem Stoffstrang ausgebildet ist, wobei der Stoffstrang zumindest bereichsweise einen Verstärkungsbereich aufweist.

Die vorliegende Erfindung betrifft auch eine Kopfbänderung für ein Patienteninterface wobei der Stoffstrang eine Breite eine Länge und eine Dicke aufweist, wobei die Breite immer größer ist als die Dicke.

Die vorliegende Erfindung betrifft auch eine Kopfbänderung für ein Patienteninterface wobei der Stoffstrang eine Breite eine Länge und eine Dicke aufweist, wobei der Verstärkungsbereich eine Verstärkung derart bewirkt, dass der Stoffstrang in Richtung der Breite weniger beweglich ist als in Richtung der Dicke.

Die vorliegende Erfindung betrifft auch eine Kopfbänderung für ein Patienteninterface wobei der Stoffstrang eine Breite eine Länge und eine Dicke aufweist, wobei der Verstärkungsbereich insbesondere entlang der Breite und/oder Länge ausgebildet ist.

Es wird offenbart eine Kopfbänderung für ein Patienteninterface wobei die Kopfbänderung aus zumindest einem Stoffstrang ausgebildet ist und einen ersten Seitenabschnitt, einen zweiten Seitenabschnitt und einen Basisabschnitt aufweist, wobei der erste Seitenabschnitt und der zweite Seitenabschnitt der Kopfbänderung jeweils zur Verbindung mit dem Patienteninterface ausgebildet sind und der Basisabschnitt den ersten Seitenabschnitt und den zweiten Seitenabschnitt miteinander verbindet.

Es wird offenbart eine Kopfbänderung für ein Patienteninterface mit einer Maske mit einem Maskenkörper und einem Maskenwulst, wobei die Kopfbänderung aus zumindest einem Stoffstrang ausgebildet ist und einen ersten Seitenabschnitt, einen zweiten Seitenabschnitt und einen Basisabschnitt aufweist, wobei sich der erste Seitenabschnitt und der zweite Seitenabschnitt der Kopfbänderung jeweils von der Maske ausgehend erstrecken und der Basisabschnitt den ersten Seitenabschnitt und den zweiten Seitenabschnitt miteinander verbindet. Der erste Seitenabschnitt und der zweite Seitenabschnitt können jeweils einen einzelnen Stoffstrang umfassen. In einer alternativen Ausgestaltung können der erste Seitenabschnitt und der zweite Seitenabschnitt jeweils mindestens zwei Stoffstränge umfassen. Der erste Seitenabschnitt und der zweite Seitenabschnitt sind in der Regel zwischen dem Basisabschnitt und dem Maskenkörper des Patienteninterface angeordnet, wobei der erste Seitenabschnitt und der zweite Seitenabschnitt jeweils eingerichtet sind, an dem Patienteninterface bzw. dem Maskenkörper des Patienteninterface befestigbar zu sein. Der Basisabschnitt ist in der Regel eingerichtet, einen hinteren Bereich eines Patientenkopfes aufzunehmen. Aufzunehmen bedeutet dabei, dass der Basisabschnitt bzw. der Stoffstrang im Bereich des Basisabschnittes derart ausgebildet ist, dass er an eine Kopfform eines Patienten anpassbar ist. Optional umfasst die Kopfbänderung zumindest zwei, in der Regel drei Stoffstränge. Dies bietet den Vorteil, dass beispielsweise der Basisabschnitt einen Stoffstrang umfasst, der erste Seitenabschnitt mindestens einen Stoffstrang, optional zwei Stoffstränge, umfasst und der zweite Seitenabschnitt ebenfalls mindestens einen Stoffstrang, optional zwei Stoffstränge, umfasst.

In einer Weiterbildung weist der Stoffstrang zumindest bereichsweise eine Materialzusammensetzung aufweist, die zumindest eine erste Stoffschicht und eine zweite Stoffschicht umfasst, wobei zwischen der ersten Stoffschicht und der zweiten Stoffschicht eine Zwischenschicht ausgebildet ist, wobei die Zwischenschicht als Schaumschicht ausgebildet ist. In der Regel wird die Schaumschicht durch die erste Stoffschicht und die zweite Stoffschicht bedeckt oder ummantelt. Die erste Stoffschicht weist in der Regel andere Eigenschaften auf, als die zweite Stoffschicht. In der Regel ist zumindest eine der ersten oder der zweiten Stoffschicht aus einem hautfreundlichen Material ausgebildet. Optional ist die jeweils andere Stoffschicht aus einem Material ausgebildet ist, dass für die Umsetzung eines Klettverschlusses geeignet ist. Die Schaumschicht weist in der Regel eine Schichtdicke zwischen 1 mm bis 5 mm, insbesondere zwischen 2 mm bis 4 mm auf.

In Ausgestaltung umfasst die Schaumschicht zumindest bereichsweise Kurzfasern und/oder Langfasern, wobei die Kurzfasern zumindest eine Ebene in der Schaumschicht ausbilden oder sich über die gesamte Schaumschicht verteilen, wobei die Kurzfasern und/oder Langfasern in einer Verbundstruktur ausgebildet sind. Kurzfasern weisen eine Länge von 0,1 mm bis 1 mm auf. Langfasern weisen eine Länge von 1 mm bis 50 mm auf. Die Kurzfasern und/oder die Langfasern können geordnet oder ungeordnet in der Schaumschicht angeordnet sein. Optional können die Langfasern gebrochen sein bzw. in kleinere Stücke zerkleinert sein.

In der Regel sind die Kurzfasern und/oder Langfasern zusammen in einer Verbundstruktur in der Schaumschicht ausgebildet. Die Verbundstruktur kann beispielsweise aus einem Basismaterial, insbesondere einer Polyurethan-Basis, bestehen, in die Kurzfasern oder Langfasern oder eine Kombination von Kurz- und Langfasern eingebracht sind. Typischerweise wird die Verbundstruktur in einem Herstellungsschritt zusammen mit der Schaumschicht hergestellt. Beispielsweise können bei der Herstellung der Schaumschicht über einen Mischkopf Kurzfasern und/oder Langfasern mit dem Basismaterial gemischt werden und direkt in die Schaumschicht eingebracht werden. Durch die direkte Einbringung der Kurzfasern und/oder Langfasern in der Verbundstruktur in die Schaumschicht wird eine Verstärkung der Schaumschicht erzielt. Ein derartig verstärkter Bereich des Stoffstrangs kann als Verstärkungsbereich bzw. als Versteifungsbereich bezeichnet werden.

In einer alternativen Ausgestaltung umfasst der Stoffstrang zumindest eine Strukturmatte. Die Strukturmatte ist als Verstärkungsmatte oder als Trennmatte ausgebildet. Optional ist die Strukturmatte eingerichtet, zumindest eine Stoffschicht von der Schaumschicht zumindest bereichsweise zu trennen. Ein Stoffstrang mit zumindest einer Strukturmatte bildet einen Verstärkungsbereich aus. Optional umfasst der Stoffstrang bereichsweise zumindest eine Strukturmatte. Dies bietet den Vorteil, dass eine Verstärkung der Kopfbänderung optional nur in den Bereichen umgesetzt werden kann, in denen eine Verstärkung benötigt wird.

In einer alternativen Ausgestaltung ist die zumindest eine Strukturmatte in die Zwischenschicht integriert ausgebildet. Integriert kann dabei beispielsweise bedeuten, dass die Strukturmatte vollständig von der Zwischenschicht bzw. der Schaumschicht umgeben ist.

Beispielsweise kann die Strukturmatte mittig in Bezug zu einer Schichtdicke der Zwischenschicht angeordnet sein. Diese Ausgestaltung bietet den Vorteil, dass die Strukturmatte bereits während des Herstellungsprozess der Zwischenschicht in die Zwischenschicht integrierbar ist und stabil mit der Zwischenschicht verbunden ist.

In einer Weiterbildung ist die Schaumschicht auf die Strukturmatte aufgetragen ausgebildet. Dabei kann die Strukturmatte als Basis für die Schaumschicht eingerichtet und ausgebildet sein. Bei der Herstellung der Schaumschicht kann der Schaum der Schaumschicht sowohl auf die Strukturmatte als auch durch die Struktur der Strukturmatte hindurch aufgebracht bzw. in die Struktur der Strukturmatte eingebracht werden. Dadurch wird die Strukturmatte bereits bei der Herstellung der Schaumschicht in diese integriert.

In einer alternativen Weiterbildung ist die zumindest eine Strukturmatte zwischen der Zwischenschicht und der ersten Stoffschicht und/oder zwischen der Zwischenschicht und der zweiten Stoffschicht angeordnet. Die Zwischenschicht bzw. Schaumschicht weist eine erste Seite und eine zweite Seite auf, wobei die erste Seite zu der ersten Stoffschicht weist und die zweite Seite zu der zweiten Stoffschicht weist. Bei der Herstellung der Schaumschicht kann somit direkt in einem weiteren Schritt nach der Ausbildung der Schaumschicht, die Strukturmatte auf die erste Seite der Schaumschicht und/oder die zweite Seite der Schaumschicht aufgebracht werden.

In einer Weiterbildung sind zumindest zwei unterschiedliche Strukturmatten in oder an der Zwischenschicht angeordnet. In eine Ausführungsform ist zumindest eine Strukturmatte an der ersten Seite der Schaumschicht ausgebildet und zumindest eine Strukturmatte an der zweiten Seite der Schaumschicht ausgebildet. Optional sind die Strukturmatte, die an der ersten Seite der Schaumschicht ausgebildet ist und die Strukturmatte, die an der zweiten Seite der Schaumschicht ausgebildet ist, unterschiedlich. Unterschiedlich bedeutet dabei, dass die Strukturmatten unterschiedliche Materialien umfassen können, unterschiedliche Strukturen aufweisen können, durch unterschiedliche Herstellungsprozesse hergestellt worden sein können und/oder unterschiedliche Dicken aufweisen können.

In Ausgestaltung ist die Strukturmatte als Gewebematte, Kettengewirk, Gestrick oder Geflecht ausgebildet. In der Regel weist bei der Ausgestaltung des Stoffstrangs mit zwei Strukturmatten jeweils eine Strukturmatte eine andere Struktur auf als die jeweils andere Strukturmatte. Beispielsweise kann eine erste Strukturmatte ein Gewebe sein, während eine zweite Strukturmatte ein Geflecht oder ein Gestrick ist.

In weiterer Ausgestaltung umfasst der Stoffstrang Kurzfasern und/oder Langfasern und zumindest eine Strukturmatte. Diese Ausgestaltung bietet den Vorteil einer Kombination zweier Verstärkungsmöglichkeiten und zu einer Ausbildung eines starken Verstärkungsbereichs. Zum einen können die Kurzfasern und/oder Langfasern zur Verstärkung in die Schaumschicht eingebracht werden. Zum anderen kann der Stoffstrang zusätzlich durch zumindest eine Strukturmatte verstärkt werden. Die Strukturmatte kann dabei in die Schaumschicht integriert ausgebildet sein oder zwischen der ersten Stoffschicht und der Schaumschicht oder zwischen der zweiten Stoffschicht und der Schaumschicht ausgebildet sein.

In einer Weiterbildung weist der Stoffstrang im Bereich des Basisabschnitts eine andere Zusammensetzung auf, als im Bereich der Seitenabschnitte. Somit kann der Stoffstrang bereichsweise eine Verstärkung durch Kurzfasern und/oder Langfasern oder durch zumindest eine Strukturmatte aufweisen. Beispielsweise kann der Stoffstrang im Bereich des Basisabschnitts einen Verstärkungsbereich aufweisen während die Seitenabschnitte keine Verstärkungsbereiche aufweisen. Alternativ können die Seitenabschnitte Verstärkungsbereiche aufweisen während der Basisabschnitt keinen Verstärkungsbereich aufweist. In der Regel weisen sowohl der Basisbereich als auch die Seitenabschnitte zumindest bereichsweise Verstärkungsbereiche auf.

In Ausgestaltung sind bzw. umfassen die Kurzfasern und/oder Langfasern und/oder die mindestens eine Strukturmatte anorganische oder organische Verstärkungsfasern. Anorganische Verstärkungsfasern können dabei Basaltfasern, Borfasern, Glasfasern, Keramikfasern, Kieselsäurefasern, Kohlenstofffasern, Quarzfasern sowie Stahlfasern sein.

Organische Verstärkungsfasern können Aramidfasern, Kohlenstofffasern, Poly(p-phenylen- 2,6-benzobisoxazol) (PBO)-Fasern, Polyester-Fasern, Nylon-Fasern, Polyethylen-Fasern oder Polymethylmethacrylat-Fasern sein. Organische Verstärkungsfasern können auch Naturfasern wie Flachs-Fasern, Hanf-Fasern, Holzfasern oder Sisalfasern sein. In der Regel sind die Kurzfasern und/oder Langfasern Naturfasern. Alternativ sind die Kurzfasern/oder Langfasern Kunststofffasern.

Die Erfindung betrifft des Weiteren ein Patienteninterface umfassend eine voranstehend beschriebene Kopfbänderung. Das Patienteninterface umfasst in der Regel eine Maske mit einem Maskenkörper, einem Maskenwulst und die Kopfbänderung. Das erfindungsgemäße Patienteninterface bietet den Vorteil, dass die Maske ohne Stirnstütze in ihrer Position gehalten werden kann. Durch die erfindungsgemäße Kopfbänderung, die zumindest bereichsweise einen voranstehend beschriebenen Stoffstrang umfasst, weist die Kopfbänderung zumindest bereichsweise eine Verstärkung auf, die die Maske im Gesicht bzw. am Kopf des Patienten abstützen und halten kann.

Im Folgenden werden anhand von stark vereinfachten schematischen Darstellungen bevorzugte Ausführungsbeispiele der Erfindung näher erläutert. Es zeigt:
Fig. 1 eine perspektivische Draufsicht auf einen Stoffstrang einer erfindungsgemäßen Kopfbänderung,
Fig. 2 eine Seitenansicht auf den Aufbau des - in Fig. 1 - gezeigten Stoffstrang,
Fig. 3 eine Seitenansicht auf einen Aufbau eines Stoffstrangs der erfindungsgemäßen Kopfbänderung mit einer Zwischenschicht mit Kurzfasern,
Fig. 4 eine Seitenansicht auf einen Aufbau des Stoffstrangs der erfindungsgemäßen Kopfbänderung mit einer Zwischenschicht mit Langfasern,
Fig. 5 eine Seitenansicht auf einen Aufbau des Stoffstrangs der erfindungsgemäßen Kopfbänderung mit einer Zwischenschicht und einer Strukturmatte zwischen einer ersten Stoffschicht und der Zwischenschicht,
Fig. 6 eine Seitenansicht auf einen Aufbau des Stoffstrangs der erfindungsgemäßen Kopfbänderung mit einer Zwischenschicht und einer in die Zwischenschicht integrierten Strukturmatte,
Fig. 7 eine Ausführungsform eines Patienteninterface mit einer Kopfbänderung in angelegtem Zustand,
Fig. 8 eine weitere Ausführungsform des Patienteninterface mit einer erfindungsgemäßen Kopfbänderung in angelegtem Zustand,
Fig. 9 eine weitere Ausführungsform des Patienteninterface mit einer Kopfbänderung in angelegtem Zustand,
Fig. 10 eine weitere Ausführungsform des Patienteninterface mit einer Kopfbänderung in angelegtem Zustand,
Fig. 11 eine Ausführungsform der Kopfbänderung mit einem Verstärkungsbereich.

In den Figuren weisen dieselben konstruktiven Elemente jeweils dieselben Bezugsziffern auf.

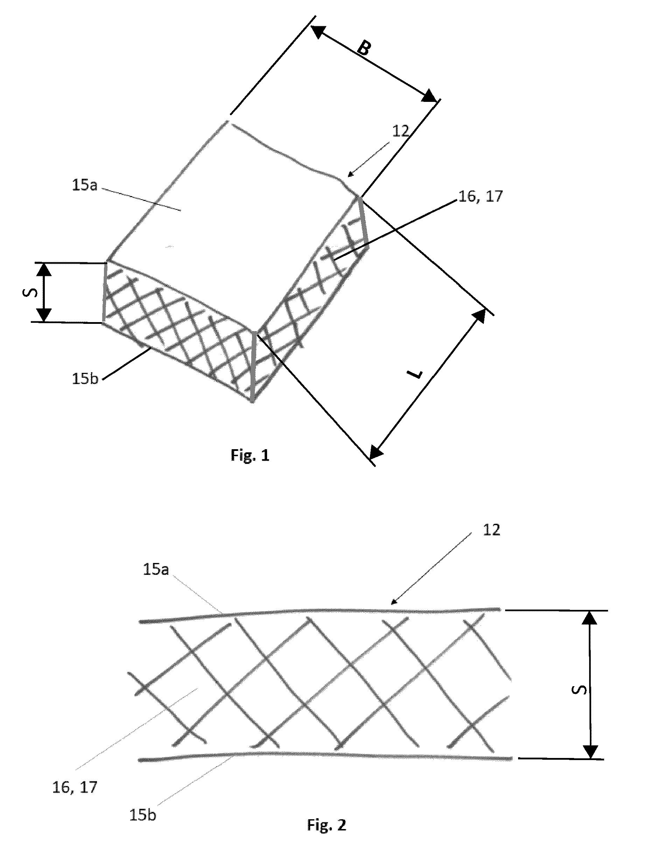

Figur 1 zeigt eine perspektivische Draufsicht auf einen Stoffstrang 12 einer erfindungsgemäßen Kopfbänderung 10. Der Stoffstrang 12 weist eine Breite (B), eine Länge (L) und eine Dicke (S) auf. Üblicherweise ist die Breite (B) immer größer ist als die Dicke (S). Die Länge ist üblicherweise größer als die Breite. Der Stoffstrang 12 weist beispielsweise einen Verstärkungsbereich auf. Gezeigt ist dabei eine erste Stoffschicht 15a und eine zweite Stoffschicht 15b, wobei zwischen der ersten Stoffschicht 15a der zweiten Stoffschicht 15b eine Zwischenschicht 16 ausgebildet ist. Die Zwischenschicht 16 macht beispielsweise einen Großteil der Dicke (S) aus. Die Zwischenschicht 16 ist in der Regel beispielsweise als Schaumschicht 17 ausgebildet.

Figur 2 zeigt eine Seitenansicht auf den Aufbau des - in Figur 1 gezeigten - Stoffstrangs 12. Gezeigt ist die erste Stoffschicht 15a und die zweite Stoffschicht 15b, wobei zwischen der ersten Stoffschicht 15a und der zweiten Stoffschicht 15b die Zwischenschicht 16, die Schaumschicht 17, ausgebildet ist. Der Stoffstrang 12 weist eine Breite (B), eine Länge (L) und eine Dicke (S) auf. Üblicherweise ist die Breite (B) immer größer ist als die Dicke (S). Die Länge ist üblicherweise größer als die Breite. Der Stoffstrang 12 weist beispielsweise einen Verstärkungsbereich auf.

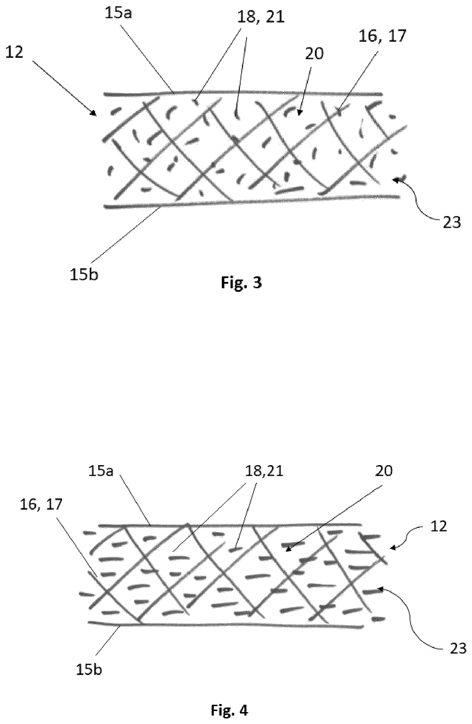

Figur 3 zeigt eine Seitenansicht einer Ausführungsform eines Aufbaus eines Stoffstrangs 12 der erfindungsgemäßen Kopfbänderung 10 mit einer Zwischenschicht 16 mit Kurzfasern 18. Gezeigt sind die erste Stoffschicht 15a und die zweite Stoffschicht 15b, wobei zwischen der ersten Stoffschicht 15a und der zweiten Stoffschicht 15b die Zwischenschicht 16, beispielsweise als Schaumschicht 17, ausgebildet ist. In der Zwischenschicht 16 sind die Kurzfasern 18 angeordnet.

In der vorliegenden Ausführungsform sind die Kurzfasern 18 dabei ungeordnet in der gesamten Schaumschicht 17 verteilt angeordnet. In einer alternativen Ausführung können die Kurzfasern 18 geordnet in der Schaumschicht 17 angeordnet sein. Insbesondere können die Kurzfasern 18 in Richtung der Breite B und/oder in Richtung der Länge L angeordnet sein. Die Kurzfasern 18 sind in der Regel in einer Verbundstruktur 20 ausgebildet. Die Verbundstruktur 20 umfasst dabei in der Regel ein Basismaterial, in das die Kurzfasern 18 eingebracht sind. In der Regel wird die Verbundstruktur 20 bei der Herstellung der Schaumschicht 17 in die Schaumschicht 17 eingebracht. In einer alternativen Ausführungsform können die Kurzfasern 18 nur bereichsweise auf oder in der Schaumschicht 17 ausgebildet sein. Die Kurzfasern 18 können auch als Langfasern 21 ausgebildet sein. Die Schaumschicht 17 und die Kurzfasern 18 in der Verbundstruktur 20 bilden einen Verstärkungsbereich 23 aus.

Figur 4 zeigt eine Seitenansicht auf eine Ausführungsform eines Aufbaus des Stoffstrangs 12 der erfindungsgemäßen Kopfbänderung 10 mit einer Zwischenschicht 16 mit Langfasern 21. Gezeigt ist die erste Stoffschicht 15a und die zweite Stoffschicht 15b, wobei zwischen ersten Stoffschicht 15a und der zweiten Stoffschicht 15b die Zwischenschicht 16, die Schaumschicht 17, ausgebildet ist. In der Schaumschicht 17 sind die Langfasern 21 angeordnet. In der vorliegenden Ausführungsform sind die Langfasern 21 dabei geordnet über die gesamte Fläche der Schaumschicht 17 verteilt angeordnet. Alternativ können die Langfasern 21 ungeordnet in der Schaumschicht 17 angeordnet sein. Insbesondere können die Fasern in Richtung der Breite B und/oder in Richtung der Länge L angeordnet sein.

Die Langfasern 21 sind in der Regel in der Verbundstruktur 20 ausgebildet. Die Verbundstruktur 20 umfasst dabei in der Regel das Basismaterial, in das die Langfasern 21 eingebracht sind. In der Regel wird die Verbundstruktur 20 bei der Herstellung der Schaumschicht 17 in die Schaumschicht 17 eingebracht. Die Schaumschicht 17 und die Langfasern 18 in der Verbundstruktur 20 bilden den Verstärkungsbereich 23 aus.

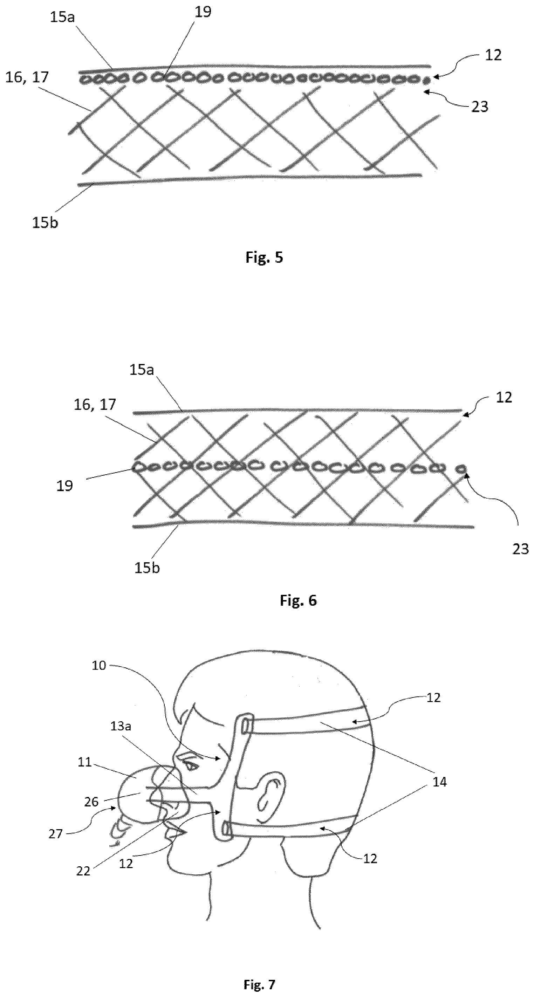

Figur 5 zeigt eine Seitenansicht auf eine Ausführungsform eines Aufbaus des Stoffstrangs 12 der erfindungsgemäßen Kopfbänderung 10 mit einer Zwischenschicht 16 und einer Strukturmatte 19 zwischen einer ersten Stoffschicht 15a und der Zwischenschicht 16. Gezeigt ist, dass die Strukturen 19 in einer Ebene zwischen der Zwischenschicht 16 bzw. der Schaumschicht 17 und der ersten Stoffschicht 15a ausgebildet sind. Alternativ kann die Strukturmatte 19 zwischen der zweiten Stoffschicht 15b und der Zwischenschicht 16 bzw. der Schaumschicht 17 angeordnet sein. In einer alternativen Ausführungsform können sowohl zwischen der ersten Stoffschicht 15a und der Zwischenschicht 17 sowie zwischen der zweiten Stoffschicht 15b und der Zwischenschicht 17 jeweils zumindest eine Strukturmatte 19 angeordnet sein. Die Schaumschicht 17 und die Strukturmatte 19 auf der Schaumschicht 17 bilden den Verstärkungsbereich 23 aus. Die Strukturmatte 19 erstreckt sich beispielsweise entlang der Länger und/oder der Breite.

Figur 6 zeigt eine Seitenansicht auf einen Aufbau des Stoffstrangs 12 der erfindungsgemäßen Kopfbänderung 10 mit einer Zwischenschicht 17 und einer in die Zwischenschicht 17 integrierten Strukturmatte 19. Gezeigt ist, dass die Strukturmatten 19 mittig integriert in der Schaumschicht 16 bzw. der Zwischenschicht 17 angeordnet ist. Alternativ kann die Strukturmatte 19 an jeder Position in der Zwischenschicht 17, der Schaumschicht 16, angeordnet sein. In einer alternativen Ausführungsform können mindestens zwei Strukturmatten 19 in der Zwischenschicht 17 angeordnet sein. Die Schaumschicht 17 und die Strukturmatte 19 in der Schaumschicht 17 bilden den Verstärkungsbereich 23 aus. Die Strukturmatte 19 erstreckt sich beispielsweise entlang der Länger und/oder der Breite.

Figur 7 zeigt eine Ausführungsform eines Patienteninterfaces 11 mit einer Maske 27 mit einem Maskenkörper 26 und einem Maskenwulst 22 und einer Kopfbänderung 10 in angelegtem Zustand. Dabei ist die erfindungsgemäße Kopfbänderung 10 gezeigt, umfassend einen ersten Seitenabschnitt 13a sowie einen Basisabschnitt 14, wobei der Basisabschnitt 14 zwei Stoffstränge 12 umfasst. Der erste Seitenabschnitt 13a verbindet das Patienteninterface 11, insbesondere den Maskenkörper 26 der Maske 27, mit dem Basisabschnitt 14 der Kopfbänderung 10. Sowohl der erste Seitenabschnitt 13a als auch ein - nicht gezeigter - zweiter Seitenabschnitt 13b und der Basisabschnitt 14 können zumindest bereichsweise einen - in den Figuren 1 bis 6 beschriebenen Stoffstrang 12 umfassen, der zumindest bereichsweise einen Verstärkungsbereich umfasst.

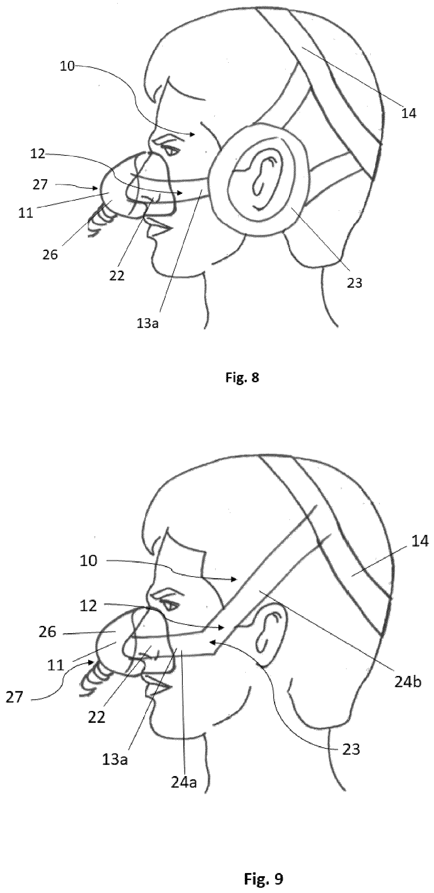

Figur 8 zeigt eine weitere Ausführungsform des Patienteninterfaces 11 mit einer Maske 27 mit einem Maskenkörper 26 und einem Maskenwulst 22 und einer erfindungsgemäßen Kopfbänderung 10 in angelegtem Zustand. Dabei ist die erfindungsgemäße Kopfbänderung 10 gezeigt, umfassend den ersten Seitenabschnitt 13a sowie den Basisabschnitt 14. Der erste Seitenabschnitt 13a weist dabei einen kreisförmigen Verstärkungsbereich 23 auf, wobei ein erster Stoffstrang 12 des ersten Seitenabschnitts 13a zu dem Maskenkörper 26 des Patienteninterface 11 geführt ist und ein zweiter Stoffstrang 12 von dem Verstärkungsbereich 23 zu dem Basisabschnitt 14 geführt ist. Alternativ kann der Verstärkungsbereich 23 oval oder eckig ausgebildet sein oder jede geometrische Form aufweisen. Der Verstärkungsbereich 23 umfasst somit einen - in den Figuren 1 bis 6 - beschriebenen Stoffstrang 12. Der Verstärkungsbereich 23 ist als Versteifung ausgebildet.

Figur 9 zeigt eine weitere Ausführungsform des Patienteninterfaces 11 mit einer Maske 27 mit einem Maskenkörper 26 und einem Maskenwulst 22 und einer Kopfbänderung 10 in angelegtem Zustand. Gezeigt ist der erste Seitenabschnitt 13a sowie der Basisabschnitt 14. Der erste Seitenabschnitt 13a weist dabei einen ersten Teil 24a und einen zweiten Teil 24b auf, wobei der zweite Teil 24b in einem Winkel zwischen 90° und 180°, insbesondere zwischen 45° und 135° zu dem ersten Teil 24a angeordnet ist. In der Regel weist der nicht gezeigte zweite Seitenabschnitt 13b dieselbe Ausgestaltung auf. Der erste Seitenabschnitt 13a weist in der Regel zumindest einen Verstärkungsbereich 23 auf, der in der Regel in einem Kontaktbereich des ersten Teils 24a und des zweiten Teils 24b ausgebildet ist.

Der erste Seitenabschnitt 13a und der Basisabschnitt 14 sind aus dem erfindungsgemäßen Stoffstrang 12 ausgebildet, wobei der Stoffstrang 12 zumindest bereichsweise, insbesondere im Bereich des ersten Seitenabschnitts 13a und/oder des zweiten Seitenabschnitts 13b, den Verstärkungsbereich 23 aufweist. Gezeigt ist auch, dass der erste Seitenabschnitt 13a an dem Maskenkörper 26 des Patienteninterfaces 11 angeordnet ist. Der zweite Seitenabschnitt 13b verbindet den ersten Seitenabschnitt 13a mit dem Basisabschnitt 14. Der Basisabschnitt 14 ist in der Regel im Wesentlichen kreisförmig ausgebildet und eingerichtet, einen Patientenkopf zumindest teilweise aufzunehmen.

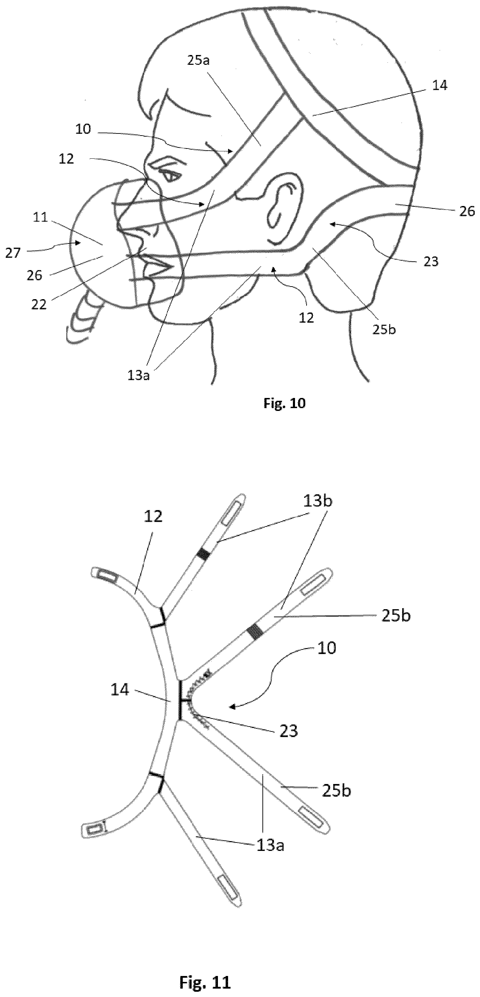

Figur 10 zeigt eine weitere Ausführungsform des Patienteninterface 11 mit einer Maske 27 mit einem Maskenkörper 26 und einem Maskenwulst 22 und einer Kopfbänderung 10 in angelegtem Zustand. Gezeigt ist der erste Seitenabschnitt 13a sowie der Basisabschnitt 14. Der erste Seitenabschnitt 13a ist aus zwei Stoffsträngen 12 ausgebildet. Ein oberer Stoffstrang 25a verbindet dabei den Maskenkörper 26 des Patienteninterface 11 mit dem Basisabschnitt 14. Der untere Stoffstrang 25b verbindet ebenfalls den Maskenkörper 26 des Patienteninterface 11 mit dem Basisabschnitt 14, wobei der untere Stoffstrang 25b getrennt von dem oberen Stoffstrang 25a ausgebildet ist. Der obere Stoffstrang 25a weist eine gebogene Form auf, wobei der obere Stoffstrang 25a zumindest bereichsweise einen Verstärkungsbereich 23 aufweisen kann. Der untere Stoffstrang 25b weist einen gewölbten Bereich 26 auf. Der gewölbte Bereich 26 wird in der Regel von dem unteren Stoffstrang 25b des ersten Seitenabschnitts 13 a und des unteren Stoffstrangs 25b des zweiten Seitenabschnitts 13b gebildet. In der Regel umfasst der gewölbte Bereich 26 einen der in den Figuren 1 bis 6 gezeigten Stoffstränge 12, der einen Verstärkungsbereich 23 ausbildet, auf.

Figur 11 zeigt die - in der Figur 10 gezeigte - Ausführungsform der Kopfbänderung 10 mit dem Verstärkungsbereich 23. Die erfindungsgemäße Kopfbänderung 10 umfasst den Stoffstrang 12 mit der erfindungsgemäßen Zusammensetzung. Die Kopfbänderung 10 weist den Basisabschnitt 14 auf, sowie den ersten Seitenabschnitt 13a und den zweiten Seitenabschnitt 13b. Der untere Stoffstrang 25b weist dabei in dem gewölbten Bereich 26 einen Verstärkungsbereich 23 auf. Der Verstärkungsbereich 23 in dem gewölbten Bereich 26 des unteren Stoffstrangs 25b bietet den Vorteil, dass bei einem Anlegen der Kopfbänderung 10, bei dem die Kopfbänderung 10 auseinandergezogen wird, der gewölbte Bereich 26 in dem Verstärkungsbereich 23 starrer ausgebildet ist, als der umgebende Stoffstrang 12.

### Bezugszeichenliste

- 10: Kopfbänderung
- 11: Patienteninterface
- 12: Stoffstrang
- 13a: erster Seitenabschnitt
- 13b: zweiter Seitenabschnitt
- 14: Basisabschnitt
- 15a: erste Stoffschicht
- 15b: zweite Stoffschicht
- 16: Zwischenschicht
- 17: Schaumschicht
- 18: Kurzfasern
- 19: Strukturmatte
- 20: Verbundstruktur
- 21: Langfasern
- 22: Maskenwulst
- 23: Verstärkungsbereich
- 24a: erster Teil
- 24h: zweiter Teil
- 25a: oberer Stoffstrang
- 25b: unterer Stoffstrang
- 26: Maskenkörper
- 27: Maske

## Patentansprüche

1. Kopfbänderung (10) für ein Patienteninterface (11) mit einer Maske (27) mit einem Maskenkörper (26) und einem Maskenwulst (22), wobei die Kopfbänderung (10) aus mehreren Stoffsträngen (12) ausgebildet ist und einen ersten Seitenabschnitt (13a), einen zweiten Seitenabschnitt (13b) und einen Basisabschnitt (14) aufweist, wobei sich der erste Seitenabschnitt (13a) und der zweite Seitenabschnitt (13b) im angelegten Zustand der Kopfbänderung (10) jeweils von der Maske (27) ausgehend erstrecken und der Basisabschnitt (14) den ersten Seitenabschnitt (13a) und den zweiten Seitenabschnitt (13b) miteinander verbindet, wobei der erste Seitenabschnitt (13a) und der zweite Seitenabschnitt (13b) im angelegten Zustand der Kopfbänderung (10) ferner zwischen dem Basisabschnitt (14) und dem Maskenkörper (26) angeordnet und jeweils an dem Patienteninterface (11) oder dem Maskenkörper (26) befestigt sind, **dadurch gekennzeichnet, dass** jeder Seitenabschnitt (13a, 13b) einen kreisförmigen oder ovalen Verstärkungsbereich (23) zum Umgeben eines Ohrs aufweist, wobei der Verstärkungsbereich (23) als Versteifung ausgebildet ist und einen der Stoffstränge (12) umfasst, wobei im angelegten Zustand der Kopfbänderung (10) ein erster Stoffstrang (12) des jeweiligen Seitenabschnitts (13a, 13b) zum Maskenkörper (26) geführt ist und ein zweiter Stoffstrang (12) des jeweiligen Seitenabschnitts (13a, 13b) vom jeweiligen Verstärkungsbereich (23) zum Basisabschnitt (14) geführt ist, und wobei auch der Basisabschnitt (14) einen Verstärkungsbereich aufweist

2. Kopfbänderung (10) nach Anspruch 1,
wobei der Verstärkungsbereich (23) auf dem Stoffstrang (12) befestigt ist.

3. Kopfbänderung (10) nach Anspruch 1 oder 2,
wobei der Verstärkungsbereich (23) in den Stoffstrang (12) integriert ist.

4. Kopfbänderung (10) nach einem der vorstehenden Ansprüche,
wobei der Verstärkungsbereich (23) Kurzfasern (18) und/oder Langfasern (21) und/oder eine Strukturmatte (19) und/oder eine Verbundstruktur (20) aufweist.

5. Kopfbänderung (10) nach einem der vorstehenden Ansprüche,
wobei der Stoffstrang (12) eine Breite (B), eine Länge (L) und eine Dicke (S) aufweist, wobei die Breite (B) immer größer als die Dicke (S) ist.

6. Kopfbänderung (10) nach einem der vorstehenden Ansprüche,
wobei der Stoffstrang (12) eine Breite (B), eine Länge (L) und eine Dicke (S) aufweist, wobei der Verstärkungsbereich (23) eine Verstärkung derart bewirkt, dass der Stoffstrang (12) in Richtung der Breite (B) weniger beweglich als in Richtung der Dicke (S) ist.

7. Kopfbänderung (10) nach einem der vorstehenden Ansprüche,
wobei der Stoffstrang (12) eine Breite (B), eine Länge (L) und eine Dicke (S) aufweist, wobei der Verstärkungsbereich (23) entlang der Breite (B) und/oder der Länge (L) ausgebildet ist.

8. Kopfbänderung (10) nach einem der vorstehenden Ansprüche,
wobei der Basisabschnitt (14) im Wesentlichen kreisförmig ausgebildet und eingerichtet ist, einen Patientenkopf zumindest teilweise aufzunehmen.

## Claims

1. A head harness (10) for a patient interface (11), comprising a mask (27) with a mask body (26) and a mask cushion (22), wherein the head harness (10) is formed from multiple fabric strands (12) and comprises a first lateral portion (13a), a second lateral portion (13b), and a base portion (14), wherein the first lateral portion (13a) and the second lateral portion (13b), in the applied state of the head harness (10), in each case extend proceeding from the mask (27) and the base portion (14) connects the first lateral portion (13a) and the second lateral portion (13b) to one another, wherein the first lateral portion (13a) and the second lateral portion (13b), in the applied state of the head harness (10), are also arranged between the base portion (14) and the mask body (26) and are in each case fastened to the patient interface (11) or mask body (26), **characterized in that** each lateral portion (13a, 13b) comprises a circular or oval reinforcing region (23) for surrounding an ear, wherein the reinforcing region (23) is designed as a stiffener and comprises one of the fabric strands (12), wherein, in the applied state of the head harness (10), a first fabric strand (12) of the relevant lateral portion (13a, 13b) is guided to the mask body (26) and a second fabric strand (12) of the relevant lateral portion (13a, 13b) is guided from the relevant reinforcing region (23) to the base portion (14), and wherein the base portion (14) also comprises a reinforcing region.

2. The head harness (10) according to claim 1,
wherein the reinforcing region (23) is fastened on the fabric strand (12).

3. The head harness (10) according to claim 1 or 2,
wherein the reinforcing region (23) is integrated in the fabric strand (12).

4. The head harness (10) according to one of the preceding claims,
wherein the reinforcing region (23) comprises short fibers (18) and/or long fibers (21) and/or textured matting (19) and/or a composite structure (20).

5. The head harness (10) according to one of the preceding claims,
wherein the fabric strand (12) has a width (B), a length (L), and a thickness (S), wherein the width (B) is always greater than the thickness (S).

6. The head harness (10) according to one of the preceding claims,
wherein the fabric strand (12) has a width (B), a length (L), and a thickness (S), wherein the reinforcing region (23) has a reinforcing effect such that the fabric strand (12) is less movable in the direction of the width (B) than in the direction of the thickness (S).

7. The head harness (10) according to one of the preceding claims,
wherein the fabric strand (12) has a width (B), a length (L), and a thickness (S), wherein the reinforcing region (23) is formed along the width (B) and/or the length (L).

8. The head harness (10) according to one of the preceding claims,
wherein the base portion (14) is substantially circular and is designed to at least partially receive a patient's head.

## Revendications

1. Harnais de tête (10) pour une interface patient (11) comprenant un masque (27) comprenant un corps de masque (26) et un bourrelet de masque (22), dans lequel le harnais de tête (10) est formé à partir de plusieurs brins de tissu (12) et présente une première section latérale (13a), une deuxième section latérale (13b) et une section de base (14), dans lequel la première section latérale (13a) et la deuxième section latérale (13b) s'étendent respectivement à partir du masque (27) dans l'état appliqué du harnais de tête (10) et la section de base (14) relie la première section latérale (13a) et la deuxième section latérale (13b) entre elles, dans lequel la première section latérale (13a) et la deuxième section latérale (13b) sont en outre disposées entre la section de base (14) et le corps de masque (26) dans l'état appliqué du harnais de tête (10) et sont fixées respectivement à l'interface patient (11) ou au corps de masque (26), **caractérisé en ce que** chaque section latérale (13a, 13b) présente une région de renforcement (23) circulaire ou ovale destinée à entourer une oreille, dans lequel la région de renforcement (23) est conçue comme une rigidification et comporte l'un des brins de tissu (12), dans lequel, dans l'état appliqué du harnais de tête (10), un premier brin de tissu (12) de la section latérale (13a, 13b) respective est amené vers le corps de masque (26) et un deuxième brin de tissu (12) de la section latérale (13a, 13b) respective est amené vers la section de base (14) à partir de la région de renforcement (23) respective, et dans lequel la section de base (14) présente également une région de renforcement.

2. Harnais de tête (10) selon la revendication 1,
dans lequel la région de renforcement (23) est fixée sur le brin de tissu (12).

3. Harnais de tête (10) selon la revendication 1 ou 2,
dans lequel la région de renforcement (23) est intégrée dans le brin de tissu (12).

4. Harnais de tête (10) selon l'une des revendications précédentes,
dans lequel la région de renforcement (23) présente des fibres courtes (18) et/ou des fibres longues (21) et/ou une natte de structure (19) et/ou une structure composite (20).

5. Harnais de tête (10) selon l'une des revendications précédentes,
dans lequel la brin de tissu (12) présente une largeur (B), une longueur (L) et une épaisseur (S), dans lequel la largeur (B) est toujours plus grande que l'épaisseur (S).

6. Harnais de tête (10) selon l'une des revendications précédentes,
dans lequel la brin de tissu (12) présente une largeur (B), une longueur (L) et une épaisseur (S), dans lequel la région de renforcement (23) produit un renforcement de telle façon que le brin de tissu (12) est moins mobile dans la direction de la largeur (B) que dans la direction de l'épaisseur (S).

7. Harnais de tête (10) selon l'une des revendications précédentes,
dans lequel la brin de tissu (12) présente une largeur (B), une longueur (L) et une épaisseur (S), dans lequel la région de renforcement (23) est formée le long de la largeur (B) et/ou de la longueur (L).

8. Harnais de tête (10) selon l'une des revendications précédentes,
dans lequel la section de base (14) présente une forme essentiellement circulaire et est configurée pour recevoir au moins partiellement la tête d'un patient.
